# FASCICULE DE BREVET EUROPEEN

(11) **EP 1 292 696 B1**
(45) Date de publication et mention de la délivrance du brevet: **02.03.2011**
(21) Numéro de dépôt: 01958149.5
(22) Date de dépôt: 20.07.2001
(51) Int. Cl.: C12P 7/06, C12P 7/08

(54) **PROCEDE DE PRODUCTION D'ETHANOL A PARTIR DE SUBSTRATS SUCRIERS AVEC REMPLACEMENT DE LEVURES**
VERFAHREN ZUR HERSTELLUNG VON ETHANOL AUS ZUCKERSUBSTRAT MIT HEFEERSETZUNG
METHOD FOR PRODUCING ETHANOL FROM SUGAR SUBSTRATES WITH YEAST SUBSTITUTION

(30) Priorité: 01.08.2000 FR 0010113
(43) Date de publication de la demande: 19.03.2003
(73) Titulaire: Bio-Ethanol Nord Picardie, 02390 Origny-Sainte-Benoite (FR)
(72) Inventeur: ALARD, Georges, Maurice, F-02120 Guise (FR); ROUX, Philippe, Jean, F-02100 St Quentin (FR); MOURIN, Alain, Yves, Gérard, F-02240 Ribemont (FR); BRASSEUR, Luc, Robert, F-02240 Pleine Selve (FR)
(74) Mandataire: Eidelsberg, Victor Albert
(86) Numéro de dépôt international: PCT/FR2001/002377
(87) Numéro de publication internationale: WO 2002/010421

(56) Documents cités:
- CH-A- 554 414
- FR-A- 2 697 266
- FR-A- 2 789 400
- GB-A- 1 309 338
- US-A- 4 419 448
- US-A- 4 743 451
- DELIA-DUPUY M L ET AL: "Contamination par les levures Brettanomyces dans les fermentations alcooliques" M.A.N. MICROBIOLOGIE ALIMENTS NUTRITION,LE PLESSIS-ROBINSON,FR, vol. 13, 1995, pages 349-359, XP002119778 ISSN: 0759-0644 cité dans la demande

## Description

La présente invention se rapporte aux procédés de production d'éthanol à partir de substrats sucriers. Elle s'applique particulièrement à la production d'éthanol en tant que carburant, mais aussi pour les industries chimiques, pharmaceutiques et cosmétiques, et après rectification de manière à enlever les substances aromatiques, alimentaires. L'invention prend notamment comme matière de départ un substrat sucrier tel que le jus de diffusion de betteraves issu du procédé de la sucrerie.

On connaît déjà de nombreux procédés de production d'éthanol par voie fermentaire à partir de substrats sucriers.

Le principe général de ces procédés consiste à diviser en deux parties le substrat sucrier. On dilue la première partie par un diluant pour obtenir le « moût faible » et on la met en contact dans un préfermenteur avec une levure du genre *Saccharomyces* pour obtenir une suspension de levures. La proportion de diluant, qui est généralement de l'eau et/ou de la vinasse, est telle que le degré alcoolique de la suspension de levures soit inférieur à 6 % en volume, en sorte qu'une concentration trop élevée en alcool n'empêche pas les levures de se développer.

La deuxième partie du substrat sucrier est également diluée avec un diluant qui est généralement de l'eau et/ou de la vinasse mais la dilution est plus faible que pour la première partie de façon à obtenir une concentration en sucres plus élevée : c'est le « moût fort ».

On met ensuite cette suspension de levures en contact avec le moût fort dans un fermenteur pendant une durée suffisante pour obtenir un vin ayant une teneur en éthanol supérieure à un seuil donné.

Deux types d'unités peuvent être distinguées selon que la biomasse est recyclée ou non.

### Fermentation sans recyclage de levures :

De la biomasse est générée en permanence dans un préfermenteur aéré appelé cuve-mère. Par un temps de séjour suffisant pour que les levures se développent mais pas trop long, par exemple d'environ 5 à 6 heures, ou par dilution, par exemple, avec de l'eau et/ou des vinasses, le moût levuré titre environ 4 à 6 % en volume d'alcool. Il est utilisé comme levain et est injecté en pied de cuve pour la fermentation proprement dite. Cet apport représente environ 30 à 50 % du volume total de la cuve. Le volume restant est alors progressivement rempli par un moût fort. La teneur finale en alcool atteint 8 à 12% en volume (fonction de la teneur en sucres dans le jus de diffusion) en 20 à 30 h.

La productivité en éthanol d'un tel procédé est relativement faible. Elle peut être améliorée en recyclant la biomasse.

### Fermentation avec recyclage de levures :

Dans le cas du recyclage de la biomasse, la levure est concentrée sous forme de crèmes de levures en fin de fermentation. Elle est réutilisée, après un lavage, nécessaire pour diluer les non-sucres, un traitement à l'acide destiné à réduire les risques d'infections bactériennes, et une rapide régénération des levures en cuve-mère aérée.

Les deux techniques évoquées ci-dessus sont applicables à une fermentation discontinue (Batch) comme à une fermentation en continu (multiétagée).

Dans l'article de Delia-Dupuy ML et al « Contamination par les levures. Brettanomyces dans les fermentations alcooliques » M.A.N. MICROBIOLOGIE ALIMENTS NUTRITION., vol. 13, 1995, pages 349 à 359 XP002119778 Le PLESSIS-ROBINSON., FR ISSN : 0759-0644, on rappelle que la flore indigène demeure une source fréquente d'accidents de fermentation dans la fermentation alcoolique et que les contaminants les plus fréquents dans la fermentation des jus de betterave sont les levures du genre Brettanomyces. On étudie trois mécanismes susceptibles d'expliquer le déplacement de population des levures pour en exclure deux et retenir que le rôle de l'acide acétique ne suffit pas à expliquer l'inhibition observée sur Saccharomyces. On propose les règles suivantes pour éviter la contamination :
- contrôler les opérations d'aération accompagnant la propagation en cuve-mère,
- suivre attentivement la qualité de la biomasse au cours des recyclages, car ces derniers pourraient permettre aux levures Brettanomyces de « dépasser » leur phase de latence.

Ces règles se sont avérées inefficaces pour lutter contre l'apparition de contaminations levuriennes dans de nombreuses unités françaises de production d'éthanol à partir de substrats sucriers y compris à partir de jus de diffusion. Ces accidents de fermentation, de plus en plus fréquents, n'ont montré aucune corrélation entre les procédés de production, les substrats utilisés et l'apparition du contaminant. Si les conditions de production et la nature du moût varient énormément, on constate une certaine « permanence » du contaminant. Il s'agit dans tous les cas du genre Dekkera ou de la levure Brettanomyces, forme imparfaite du genre précédemment cité. Dans tous les cas, l'accident nécessite un arrêt total de l'atelier de fermentation, suivi d'un redémarrage complet après désinfection totale de l'atelier.

L'invention pallie ces inconvénients en évitant l'arrêt de l'atelier et par la même une baisse de la productivité de l'installation de fermentation. Elle permet de conduire une fermentation en continu tout en s'affranchissant des contaminations levuriennes.

L'invention a pour objet un procédé de production d'éthanol, qui consiste à mettre en contact avec une levure du genre Saccharomyces dans un préfermenteur du jus de diffusion de sucrerie soit pendant une durée suffisamment faible, notamment pendant 5 à 6 heures (fonction de la concentration en sucres dans le jus de diffusion), soit par dilution, par exemple, avec de l'eau et/ou des vinasses pour obtenir une suspension de levures d'un degré alcoolique inférieur à 6% en volume, puis à mettre la suspension de levures en contact avec du jus de diffusion de sucrerie pendant une durée suffisante pour obtenir un vin ayant une teneur en sucres inférieure à 3g/l, de préférence inférieure à 2 g/l et mieux inférieure à 1g/l avec un degré alcoolique du vin de 8 à 12% en volume (fonction de la teneur en sucres dans le jus de diffusion) et à distiller le vin pour obtenir de l'éthanol, le procédé étant caractérisé en ce qu'il consiste à enlever sensiblement toutes les levures présentes dans le préfermenteur et à les y remplacer par des levures fraîches à des intervalles de temps tels que la concentration en micro-organismes, autres que la levure du genre Saccharomyces, dans le préfermenteur reste inférieure à un seuil donné pendant l'intervalle suivant le remplacement des levures.

On a en effet constaté, d'une manière inattendue, que les ralentissements, notamment en installation de fermentation continue, étaient dus au fait qu'en relativement peu de temps la levure du genre Saccharomyces subit une dégénérescence, une mutation et/ou une contamination par un autre micro-organisme, notamment par une levure du genre Brettanomyces bien moins active. En remplaçant toutes les levures par des levures fraîches du genre Saccharomyces avant que ne se produise ce phénomène, et notamment de préférence avant qu'il n'y ait 10⁷ cellules par millilitre, et mieux 10⁶ cellules par millilitre, d'un microorganisme autre que la levure du genre Saccharomyces dans le préfermenteur, on maintient ainsi l'activité des levures de fermentation tout en s'affranchissant du risque de contamination levurienne. La fraîcheur de la suspension de levures joue un rôle primordial sur l'absence d'infection par des levures du genre Brettanomyces. L'apport de levures fraîches aux levures déjà contaminées ne permet pas d'éviter la contamination levurienne. Pour maintenir de manière continue l'absence des levures du genre Brettanomyces, il faut éliminer pratiquement toutes les levures anciennes avant d'introduire les nouvelles levures.

Le procédé suivant l'invention se distingue d'une technique sans préfermenteur, décrite par exemple au US-A 4 419 448, dans laquelle on vidange et nettoie les fermenteurs lorsque le milieu fermentaire est si contaminé que la centrifugation ( « Yeast separator ») n'est pas suffisamment efficace pour séparer les levures des bactéries de sorte qu'une grande quantité de microorganismes est recyclée dans le 1^{er} fermenteur en même temps que les levures. C'est pourquoi les levures ne sont plus recyclées mais envoyées en totalité à un sécheur et remplacées par des levures fraîches dans la cuve A. L'apport des levures n'est pas un moyen préventif mis en oeuvre systématiquement, mais une conséquence de l'infection des fermenteurs.

Le brevet américain propose un dispositif permettant de réduire les impacts sur l'allure de l'unité d'un traitement curatif (la vidange et le nettoyage des fermenteurs) qui nécessite un renouvellement des levures ; alors que l'invention propose une solution préventive basée sur un remplacement systématique des levures qui évite la vidange des fermenteurs (il n'y a d'ailleurs pas de fermenteur auxiliaire suivant l'invention).

Le procédé suivant l'invention se distingue aussi du procédé décrit au CH 554 414 permettant de combattre la mousse provoquée par un fort dégagement de CO₂ induit par une forte activité fermentaire liée à une forte concentration en levures dans un moût de brasserie. Ce brevet antérieur porte sur le dégazage obtenu par une extraction continue de moût en fermentation, une séparation de la phase liquide (moût) et des levures, une maturation (c'est-à-dire en fait un dégazage naturel) du moût et un recyclage des levures. Mais comme la récupération des levures n'est que partielle puisqu'une partie de celle-ci reste dans le moût, on maintient la concentration initiale en levures dans le fermenteur par un apport de levures fraîches. La levure séparée est retournée au fermenteur ce qui est contraire à la présente invention. L'apport de levures fraîches ne constitue pas un procédé permettant de pallier une infection, ni même d'améliorer la fermentation, mais un moyen de compenser la perte de levures liée au procédé de dégazage (dont la finalité est de réduire la formation de mousse. Ce brevet suisse décrit d'ailleurs un procédé de fermentation de bière complètement différent du.point de vue de la température et des temps de fermentation d'un procédé de production d'éthanol. Dans la bière, la fermentation a lieu à une température basse de 6 à 8°C avec des temps de fermentation de 10 à 12 jours ou à une température de 10 à 20° C pendant 3 à 5 jours.

Suivant la présente invention, on peut détecter la présence de Brettanomyces dans le préfermenteur en prélevant un échantillon de la suspension de levures et en l'examinant au microscope. Alors que les Saccharomyces, et notamment Saccharomyces cerevisiae, ont une forme ovoïde, les Brettanomyces ont une forme allongée. On peut donc remplacer toutes les levures dans le préfermenteur par des levures fraîches dès qu'il apparaît au microscope un micro-organisme de forme allongée. On peut également savoir par l'expérience le délai à respecter pour l'ajout des levures fraîches et remplacer systématiquement les anciennes levures par des levures du genre Saccharomyces à un intervalle de temps inférieur à 4 jours.

De préférence, on apporte des levures fraîches au préfermenteur en une quantité telle que l'on obtient dans le préfermenteur une concentration au moins égale à 10⁶ cellules par millilitre environ et de préférence 10⁷ cellules par millilitre. On permet ainsi d'éviter toute contamination levurienne en maintenant la concentration de la population souhaitée de levures du genre Saccharomyces.

De préférence, le procédé consiste à engager de 25 à 50% du poids de jus de diffusion dans le préfermenteur pour obtenir la suspension de levures et à mettre le reste du jus de diffusion en contact avec la suspension de levures.

### Préfermentation :

La préfermentation ou propagation des levures (ex : Saccharomyces cerevisiae, Saccharomyces pombe...) peut être avantageusement réalisée pour obtenir une concentration au moins égale à 10⁷ cellules par millilitre dans 4 préfermenteurs (volume des préfermenteurs : 45m³ chacun) fonctionnant en parallèle.

Le jus de diffusion ou jus de betteraves provient du résultat de l'étape de diffusion en sucrerie qui consiste à mettre en contact les cossettes (betteraves coupées en fines lamelles) avec de l'eau à une température de 65 - 80°C afin de récupérer le sucre présent dans les cossettes par phénomène d'osmose. La durée de préfermentation est calculée en divisant le volume de remplissage de moûts dans les préfermenteurs par le débit d'alimentation de ces préfermenteurs en jus de diffusion (cette durée est naturellement fonction de la teneur en sucres dans le jus de diffusion, elle est généralement de 5 à 6h).

Le jus ainsi obtenu, généralement appelé jus de diffusion, a la composition suivante en volume :

| | |
|---|---|
| Teneur en matières sèches : | 13 à 22% Saccharose 12 à 20% |
| Eau : 78 à 87 % | Non-sucre 2 à 4% |
| | |
| Matières minérales : | - Na + 80 à 120 mg/l |
| | - K + 120 à 1500 mg/l |
| | - Ca++ 30 à 70 mg/l |
| | - Mg++ 100 à 150 mg/l |
| | - Mn++ 10 à 30 mg/l |

Le jus de diffusion est distribué dans les 4 préfermenteurs et sert de substrat de croissance aux micro-organismes.

La température dans les préfermenteurs est rigoureusement contrôlée et régulée par un système de plaques de refroidissement dans lequel circule un liquide de refroidissement à l'intérieur ou à l'extérieur des cuves de préfermentation.

Toute multiplication de micro-organismes entraîne une élévation de température. Toute-variation de température peut devenir inhibitrice pour la propagation des levures.

La température dans les préfermenteurs est maintenue entre 30 et 35°C.

Afin de favoriser le développement des levures, le milieu nutritif nécessaire à la multiplication de ces micro-organismes comprend :
- des sucres fermentescibles
- de l'azote apporté sous formes diverses telles que l'urée, l'ammoniac, les sels d'ammonium
- du phosphore apporté sous formes diverses telles que l'acide phosphorique, les phosphates
- du soufre apporté sous formes diverses telles que l'acide sulfurique, les sulfates
- de l'oxygène
- des minéraux essentiels si une carence quelconque est détectée.

Ces éléments nutritifs préviennent tout ralentissement de la propagation des levures.

Ces éléments nutritifs et l'oxygène sous forme d'air comprimé (ou d'eau oxygénée) sont régulièrement fournis afin de favoriser le développement des levures et non la fermentation alcoolique.

A titre d'exemple :
- Le débit d'air dans chaque préfermenteur est d'environ 30 Nm3/h.
- 5 kg de sulfate d'ammonium ayant une teneur en azote minimale de 21 % et en eau de 0,2 % maximum (HOLVOET Chimie Chaussée de Leuze 144, Leuzesesteenweg Belgique ; INTERFERT, 28, rue d'Armenonville, 92200 Neuilly sur Seine...) et 5 kg de phosphate diammonique d'une pureté de 95 % minimum et ayant une pureté en P₂O₅ comprise entre 52 et 55 % (RHODIA Chimie, 299, rue du Président Pompidou, BP 202, 59561 La Madeleine Cedex ; PRAYON France, 80-82, rue de Paris, 93804 Epinay sur Seine Cedex) sont versés toutes les heures dans chaque préfermenteur sous forme d'une solution aqueuse.

Afin d'éviter tout risque d'infection par des micro-organismes étrangers, l'installation industrielle se trouvant en plein air ; les préfermenteurs sont nettoyés et désinfectés à tour de rôle par nettoyage à l'eau filtrée de rivière puis injection de vapeur durant une durée prédéterminée de 10 minutes minimum.

Cela permet par ailleurs d'éviter les infections par des levures dites "sauvages" et la dégénérescence progressive de la souche levurienne prédominante.

Une identification de ces micro-organismes contaminants détectés sur le site a été réalisée : il s'agit de Brettanomyces bruxellensis.

Cette levure contaminante a une forme allongée caractéristique très différente de la morphologie de la Saccharomyces cerevisiae utilisée.

Des observations microscopiques quotidiennes (microscope universel à lumière transmise de marque ZEISS à grossissement X 400) des moûts levurés ont été mises en place sur le site de fabrication et permettent de détecter instantanément l'apparition de ces levures sauvages de type Brettanomyces.

Des comptages et reconnaissances morphologiques sur boîtes de Petri viennent confirmer à posteriori les observations microscopiques.

Le milieu utilisé pour le comptage sur boîtes de Petri dénommé milieu de MALT WICKERHAM est composé, pour 2 litres de préparation, de :
- extrait de malt 3 g (référence Laboratoire Merck 105391)
- peptone 5 g (référence Laboratoire Merck 7212)
- extrait de levure 3 g (référence Laboratoire Merck 103753)
- glucose 10 g
- gélose nutritive 10 g (référence Laboratoire Merck 1614).

L'échantillon du moût levuré est dilué au dixième successivement dans du sérum physiologique (solution de NaCl à 9 pour mille) jusqu'à obtenir les dilutions suivantes : 10⁻⁵ ; 10⁻⁶ ; 10⁻⁷. 1 ml de ces dilutions est ensemencé en profondeur selon les techniques classiques de microbiologie avec le milieu de MALT WICKERHAM.

L'espacement de la fréquence de rajout de la souche souhaitée pour le processus fermentaire a pour conséquence une contamination prioritaire par l'organisme microbien indésirable entraînant une augmentation du temps de fermentation ainsi qu'une diminution de la productivité alcoolique.

Un retard dans la fréquence impérative de rajout de la souche souhaitée et de remplacement des levures anciennes entraîne par ailleurs la nécessité d'accélérer les adjonctions de levures afin d'éradiquer la persistance de la contamination.

### Procédé de remplacement des levures anciennes par des nouvelles levures

Lors de la propagation de levures fraîches, par exemple un préfermenteur est choisi pour la réalisation de cette opération, les actions à mener sont les suivantes :

Le préfermenteur étant vidangé, le laver à l'eau puis le nettoyer à la vapeur (Par exemple : Pression = 3 bars absolus, température = 130°C) selon une durée prédéterminée de 10 minutes minimum.

On peut également réaliser ce nettoyage avec de l'eau formolée (formol 30,5% : CALDIC France B.P. 722 51056 REIMS) ou tout désinfectant classique tels que :
Les familles des composés halogènes : chlore ou iode et leurs dérivés
Les agents d'oxydation (peroxyde d'hydrogène, permanganate de potassium)
Les composés aminés (Bactanios 95 employé en solution de 0,2 à 0,5 %, Anios Pavé du Moulin 59260 Lilles Hellemmes).

Les acides et alcalis forts (L'acide sulfurique concentré à 96% employé à une dilution de 5 à 10%, Tessenderlo Chemie rue du Trône 130 B Bruxelles), (La lessive de soude concentrée à 30,5 %, employée à chaud à une dilution de 1 à 2 %, CLEMENT RPC Ets LOMME Rue Pelouze, BP 117 59461 LOMME Cedex) (Agrobac employé à des dilutions de 2 à 7,5 % : MINOT APURA 88 rue de Marquillies 59044 Lilles Cedex), (Agromousse employé à des dilutions de 5 à 7%, MINOT APURA 88 rue de Marquillies 59044 Lilles Cedex). (L'Aniosteril acide désinfectant employé à des doses de 1 à 1,5% Anios Pavé du Moulin 59260 Lilles Hellemmes), (GALOR C7 employé à des doses de 1 à 5%, Anios Pavé du Moulin 59260 Lilles Hellemmes).

Les aldéhydes et tensioactifs (Anios W4 employé à 0,5 % en pulvérisation temps de contact 5 à 10 minutes ou en circulation à 0,4 % temps de contact 20 à 30 minutes, Anios Pavé du Moulin 59260 Lilles Hellemmes).

Les pourcentages indiqués ci-dessus sont exprimés en poids.

La procédure de nettoyage - désinfection peut comporter 5 phases :
- prélavage ou pré-nettoyage : élimination mécanique des souillures grossières par jet d'eau,
- nettoyage : élimination du reste des souillures à l'aide d'une solution de nettoyage,
- rinçage : élimination de la solution de nettoyage dans laquelle les souillures ont été dispersées,
- désinfection : destruction chimique de la biocontamination de surface à l'aide d'une solution désinfectante,
- rinçage final: élimination de la solution désinfectante résiduelle.

Une combinaison des opérations citées ci-dessus permet dans tous les cas d'aboutir à une désinfection suffisante du préfermenteur destiné à recevoir les levures fraîches sans contamination avec les anciennes levures.

Le préfermenteur est rempli à un tiers environ de sa capacité avec du jus de diffusion.

On régule rigoureusement la température dans le préfermenteur (inférieure à 34°C).

Les 300 kg de levures fraîches à environ 32% en poids de matières sèches sont ajoutées dans le préfermenteur.

Un apport en sels nutritifs et en air est réalisé et régulé.

L'alimentation en jus de diffusion est poursuivie tout en contrôlant la densité et la température dans le préfermenteur.

Ce dernier, une fois rempli, est mis en communication successivement avec les autres préfermenteurs préalablement vidés, nettoyés et stérilisés à la vapeur ou chimiquement de façon à ne pas contaminer les nouvelles levures par les anciennes.

On alimente ainsi progressivement les 4 préfermenteurs avec les nouvelles levures.

### Fermentation

L'objet de la présente invention est principalement applicable en fermentation dite continue, procédé par lequel les fermenteurs travaillent en cascade, c'est-à-dire que la fermentation n'est que partielle dans chaque fermenteur jusqu'au dernier où la fermentation est achevée.

L'alimentation des fermenteurs en moût levuré et jus de diffusion est réalisée en continu sur le ou les deux ou les trois premiers fermenteurs, appelés fermenteurs de tête, les fermenteurs suivants étant nommés fermenteurs de chute. Le nombre de fermenteurs de tête et la répartition des alimentations en moûts dépendent des volumes des fermenteurs.

La température dans les fermenteurs est maintenue entre 30 et 35°C.

La fermentation se poursuit ensuite en cascade dans chaque fermenteur jusqu'à ce que la teneur en sucre du vin obtenu soit inférieure à 3 g/l et, de préférence à 2 g/l et mieux à 1 g/l, pour un degré alcoolique du vin de 8 à 12% en volume (fonction de la teneur en sucres dans le jus de diffusion), le moût fermenté du dernier fermenteur est alors envoyé, toujours en continu, dans les bacs à vin, avant d'alimenter l'atelier de distillerie.

On détermine le degré alcoolique des vins par voie enzymatique à l'aide de l'analyseur biochimique YSI 2700 SELECT (ROUCAIRE, 2 av. du Pacifique BP 78 Les Ulis 91493 Courtaboeuf cedex). On effectue une dilution au 50^{ème} avec de l'eau déminéralisée de l'échantillon de vin. Cet échantillon dilué et filtré est ensuite passé dans l'analyseur biochimique YSI 2700 SELECT qui donne automatiquement la teneur en éthanol en g/l. Pour obtenir le résultat en degré alcoolique (% volume par volume), il suffit de diviser cette valeur par 7.88 après avoir pris en compte le facteur de dilution.

On détermine la teneur en glucose restant par voie enzymatique à l'aide de l'analyseur biochimique YSI 2300 STAT PLUS (ROUCAIRE, 2 av. du Pacifique BP 78 Les Ulis 91493 Courtaboeuf cedex). On effectue les dilutions nécessaires des échantillons selon la teneur supposée en glucose restant. On filtre l'échantillon et on le passe ensuite dans l'analyseur biochimique YSI 2300 STAT PLUS qui donne une teneur en glucose restant exprimée en mg/dl. Pour l'obtenir en g/l, il suffit de multiplier le résultat par 100 après avoir pris en compte le facteur de dilution éventuel.

### Production de flegmes (alcool brut)

(La) ou les colonne(s) distillatrice(s) peuvent fonctionner en parallèle ou en série (double effet) sous vide ou sous pression, sont chauffées par de la vapeur soit en injection directe, soit par l'intermédiaire de bouilleurs, soit par thermocompression.

Cette vapeur peut également chauffer les colonnes de Lütter quand elles existent, les vinasses étant généralement recyclées dans l'étape de diffusion de la sucrerie.

Le moût fermenté ou « vin » provenant de la fermentation, après passage dans un échangeur thermique, alimente la ou les colonnes distillatrices. Les vapeurs d'alcools des colonnes sont condensées dans des échangeurs thermiques.

L'alcool ou flegme est concentré à 90-96 % volume (selon les contraintes d'investissement) dans la ou les colonnes distillatrices puis refroidi dans des échangeurs avant stockage. Une extraction des impuretés volatiles peut être réalisée sur la ou les colonnes de distillation afin d'améliorer la qualité des flegmes. Ces mauvais goûts extraits et moins valorisables sont stockés dans une cuve différente.

### Exemple illustrant la présente invention

On remplace les levures anciennes à une fréquence inférieure à 4 jours par 300 kg de levures fraîches (ex : Saccharomyces cerevisiae) pressées à 32 % de matières sèches; chaque gramme de produit contient environ 10 milliards de cellules vivantes, ceci pour un jus de diffusion de betteraves à une concentration en sucres égale à 16%. L'utilisation de levures lyophilisées ou de crème concentrée commerciale de levures (Saccharomyces cerevisiae ou pombe) permet également d'aboutir au même résultat.

Le remplacement régulier des anciennes levures a permis de s'affranchir totalement des contaminations par des organismes indésirables et d'éviter ainsi un arrêt complet de l'installation, comme cela était le cas précédemment et comme cela arrive fréquemment sur d'autres unités de fabrication.

## Revendications

1. Procédé de production d'éthanol, dans lequel on met une levure du genre Saccharomyces en contact avec du jus de diffusion de sucrerie soit pendant 5 à 6 heures en fonction de la teneur en sucres dans le jus de diffusion, soit par dilution, par exemple, avec de l'eau et/ou des vinasses pour obtenir une suspension de levures d'un degré alcoolique inférieur à 6% en volume, puis on met la suspension de levure en contact avec du jus de diffusion de sucrerie pendant une durée suffisante pour obtenir un vin ayant une teneur en sucres inférieure à 3g/l, avec un degré alcoolique du vin de 8 à 12% en volume (fonction de la teneur en sucres dans le jus de diffusion) et on distille le vin pour obtenir de l'éthanol, **caractérisé en ce que** l'on met la levure en contact avec le jus de diffusion dans un préfermenteur, on enlève toutes les levures présentes dans le préfermenteur, puis on .nettoie et on désinfecte le préfermenteur et on met des levures fraîches à des intervalles de temps tels que la concentration en micro-organismes, autres que la levure du genre Saccharomyces, dans le préfermenteur reste inférieure à un seuil de 10⁷ cellules par millilitre pendant l'intervalle suivant le remplacement des levures.

2. Procédé suivant la revendication 1, **caractérisé en ce que** le seuil est de 10⁶ cellules par millilitre.

3. Procédé suivant l'une des revendications 1 ou 2, **caractérisé en ce qu'**il consiste à remplacer toutes les levures dans le préfermenteur par des levures fraîches dès qu'il apparaît au microscope un micro-organisme de forme allongée.

4. Procédé suivant l'une des revendications précédentes, **caractérisé en ce qu'**il consiste à remplacer toutes les levures du préfermenteur par des levures fraîches en une quantité telle que l'on obtient dans le préfermenteur une concentration au moins égale à 10⁶ cellules par millilitre.

5. Procédé suivant l'une des revendications précédentes, **caractérisé en ce qu'**il consiste à engager de 25 à 50% du poids de jus de diffusion dans le préfermenteur pour obtenir la suspension de levures et à mettre le reste du jus de diffusion en contact avec la suspension de levures.

6. Procédé suivant l'une des revendications précédentes, **caractérisé en ce qu'**il consiste à mettre le jus de diffusion en contact avec une levure du genre Saccharomyces à une température de 30 à 35°C.

7. Procédé suivant l'une des revendications précédentes, **caractérisé en ce qu'**il est effectué en continu.

8. Procédé suivant la revendication 4, **caractérisé en ce que** la concentration est au moins égale à 10⁷ cellules par millititre.

## Claims

1. Process for the production of ethanol in which a yeast of the genus Saccharomyces is brought into contact with raw sugar juice, either for 5 to 6 hours defending on the sugar content of the raw juice, or by dilution, e.g. with water and/or slops, to give a yeast suspension in which the percentage of alcohol is below 6 vol.%, the yeast suspension is then brought into contact with raw sugar juice for a sufficient time to give a wine in which the sugar content is less than 3 g/l and the percentage of alcohol is 8 to 12 vol.% (depending on the sugar content of the raw juice), and the wine is distilled to give ethanol, **characterized in that** the yeast is brought into contact with the raw juice in a prefermenter, all the yeasts present in the prefermenter are removed, the prefermenter is then cleaned and disinfected and fresh yeasts are placed in it at intervals of time such that the concentration of microorganisms, other than the yeast of the genus Saccharomyces, in the prefermenter remains below a threshold of 10⁷ cells per millilitre during the interval following the replacement of the yeasts.

2. Process according to Claim 1, **characterized in that** the threshold is 10⁶ cells per millilitre.

3. Process according to Claim 1 or 2, **characterized in that** it consists in replacing all the yeasts in the prefermenter with fresh yeasts as soon as a microorganism of elongate shape appears under the microscope.

4. Process according to one of the preceding claims, **characterized in that** it consists in replacing all the yeasts in the prefermenter with fresh yeasts in an amount such that a concentration equal to at least 10⁶ cells per millilitre is obtained in the prefermenter.

5. Process according to one of the preceding claims, **characterized in that** it consists in using from 25 to 50% of the weight of raw juice in the prefermenter to give the yeast suspension, and in bringing the remainder of the raw juice into contact with the yeast suspension.

6. Process according to one of the preceding claims, **characterized in that** it consists in bringing the raw juice into contact with a yeast of the genus Saccharomyces at a temperature of 30 to 35°C.

7. Process according to one of the preceding claims, **characterized in that** it is carried out continuously.

8. Process according to Claim 4, **characterized in that** the concentration is equal to at least 10⁷ cells per millilitre.

## Patentansprüche

1. Verfahren zur Herstellung von Ethanol, bei welchem eine Hefe der Gattung Saccharomyces in Kontakt mit einem Zucker-Diffusionssaft gebracht wird, sei es während 5 bis 6 Stunden in Abhängigkeit des Zuckergehalts in dem Diffusionssaft, sei es durch Verdünnen, z.B. mit Wasser und/oder mit Vinassen, um eine Suspension aus Hefen mit einem Alkoholanteil unter 6 Volumenprozent zu erhalten, dann die Hefesuspension für eine ausreichende Dauer in Kontakt mit einem Zucker-Diffusionssaft gebracht wird, um einen Wein mit einem Zuckergehalt von weniger als 3 g/l zu erhalten, mit einem Alkoholanteil im Wein von 8 bis 12 Volumenprozent (abhängig von dem Zuckergehalt in dem Diffusionssaft), und bei welchem der Wein destilliert wird, um Ethanol zu erhalten,
**dadurch gekennzeichnet, dass** die Hefe mit dem Diffusionssaft in einem Vorfermenter in Kontakt gebracht wird, alle in dem Vorfermenter vorliegenden Hefen entfernt werden, dann der Vorfermenter gereinigt und desinfiziert wird, und frische Hefen derart zu Zeitintervallen zugegeben werden, dass die Konzentration an von der Hefe der Gattung Saccharomyces abweichenden Mikroorganismen in dem Vorfermenter während dem Intervall entsprechend dem Ersetzen der Hefen unter einer Grenze von 10⁷ Zellen pro Milliliter bleibt.

2. Verfahren nach Anspruch 1, **dadurch gekennzeichnet, dass** die Grenze 10⁶ Zellen pro Milliliter beträgt.

3. Verfahren nach einem der Ansprüche 1 oder 2, **dadurch gekennzeichnet, dass** das Verfahren darin besteht, alle Hefen in dem Vorfermenter durch frische Hefen zu ersetzen, sobald am Mikroskop ein Mikroorganismus mit langgestreckter Form erscheint.

4. Verfahren nach einem der vorhergehenden Ansprüche, **dadurch gekennzeichnet, dass** das Verfahren darin besteht, alle Hefen des Vorfermenters in solcher Menge durch frische Hefen zu ersetzen, dass in dem Vorfermenter eine Konzentration von mindestens 10⁶ Zellen pro Milliliter erhalten wird.

5. Verfahren nach einem der vorhergehenden Ansprüche, **dadurch gekennzeichnet, dass** das Verfahren darin besteht, im Bereich von 25 bis 50% des Gewichts vom Diffusionssaft in den Vorfermenter einzubinden, um die Hefesuspension zu erhalten, und den Rest des Diffusionssafts in Kontakt mit der Hefesuspension zu bringen.

6. Verfahren nach einem der vorhergehenden Ansprüche, **dadurch gekennzeichnet, dass** das Verfahren darin besteht, den Diffusionssaft bei einer Temperatur von 30 bis 35 °C in Kontakt mit einer Hefe der Gattung Saccharomyces zu bringen.

7. Verfahren nach einem der vorhergehenden Ansprüche, **dadurch gekennzeichnet, dass** es im Durchlauf durchgeführt wird.

8. Verfahren nach Anspruch 4, **dadurch gekennzeichnet, dass** die Konzentration mindestens 10⁷ Zellen pro Milliliter beträgt.
